# EUROPEAN PATENT APPLICATION

(11) **EP 1 118 342 A1**
(43) Date of publication of application: **25.07.2001**
(21) Application number: 00100260.9
(22) Date of filing: 18.01.2000
(51) Int. Cl.: A61L 15/36, A61F 13/15, B32B 9/00

(54) **Articles comprising a spore-forming lactic acid-producing micro-organism**

(71) Applicant: THE PROCTER & GAMBLE COMPANY, Cincinnati, Ohio 45202 (US)
(72) Inventor: Di Cintio, Achille, 65126 Pescara (IT); Talone, Donatella, 66043 Casoli (Chieti) (IT); Pesce, Antonella, 65120 Pescara (IT)
(74) Representative: Kremer, Véronique Marie Joséphine

(57) **Abstract**

The present invention relates to articles, preferably disposable absorbent articles like sanitary napkins and pantiliners, which comprise spore-forming lactic acid-producing microorganisms, preferably the species *Lactobacillus sporogenes.* These articles have the ability to exhibit long lasting antagonistic properties against undesirable strains of microorganisms, typically long lasting odor control while providing an effective level of protection.

## Description

### Field of the Invention

This invention relates to an article, such as a disposable absorbent article, intended to be brought into contact with a user's skin, for example the skin in the perineum, this article comprises a spore-forming lactic acid-producing microorganism.

### Background of the Invention

Infections on skin, especially in the urogenital region is a problem that affects many individuals. In and around the anus, there are many different kinds of microorganisms as well as a large amount of these microorganisms. It is known that one reason for many infections in the urogenital region is that microorganisms from a person's own intestinal flora spread from the anus to urogenital organs over perineum and there cause infection.

Normally, an ecological balance prevails between different microorganisms on skin and mucus membrane, and the normal microbiological flora is highly significant in preventing the establishment of undesirable microorganisms. Lactic acid producing bacteria, play an active role in this respect. However, situations are found where this natural defense system is inadequate and is disturbed in a way that enables potentially pathogenic microorganisms to become established and give rise to the generation of infection, for instance in conjunction with medication, poor hygiene, skin changes and changes in mucus membranes.

Another problem associated with situations where the natural equilibrium is perturbated is the generation of undesirable odours, this is especially noticeable in case of vaginal discharge including vaginal fluids, and/or menstruation, urinal discharge, faeces or perspiration.

Microorganisms or their products that are known to contribute to the occurrence of undesirable odours, to cause infections in the urinal tract or to be associated with the occurrence of skin problems are for instance microorganisms from the genera Pseudomonas, Escherichia, Enterococcus, Proteus, Klebsiella, Streptococcus, Staphylococcus, Gardnerella and Candida.

Articles are available which aim to prevent the occurrence of such infections and/or to avoid and minimise the detection of malodours. For example International Patent Application WO 92/13577 describes a tampon or sanitary napkin that has been impregnated with a culture of lactic-acid producing bacteria, preferably of the genus Pediococcus, isolated from healthy individuals. The tampon or sanitary napkin is intended for the prophylactic treatment of urogenital infections. WO 97/02846 discloses an absorbent article with antagonistic microorganisms selected from the family Lactobacillaceae, preferably from the genera Lactobacillus or Lactococcus.

However, the problem encountered with such articles of the prior art is the stability of the article during use. None of these prior art documents discloses anything about providing articles which are able to deliver long lasting antagonistic properties against undesirable strains of microorganisms upon prolonged use condition, i.e., that maintain a sufficient amount of active and transferable bacteria. Consequently, there is a need for articles delivering antagonistic properties against pathogens, which articles are specially adapted for maintaining their antagonistic properties against pathogens upon prolonged use condition.

Thus the object of the present invention is to provide an article suitable for external application to a human or animal, without permitting pathogenic microorganisms to grow or to become active to an extent such as to promote undesirable odours, to incur the risk of infections and/or to have a negative effect on the skin.

More particularly it is an object of the present invention to provide articles, especially disposable absorbent articles, which deliver sustained antagonistic properties against undesirable strains of micro-organisms. It is a further object of the present invention to provide disposable absorbent articles, which deliver effective odour control over prolonged use time of the articles, i.e., a long lasting odour control by preventing the formation of odour over prolonged wearing time of the article. It is yet a further object of the present invention to provide such articles which are effectively controlling odour over a broad spectrum of malodours, mainly by preventing the formation of such malodours.

These objects have been achieved in accordance with the present invention by adding to an article, such as a disposable absorbent article, spore-forming lactic-acid producing microorganisms, preferably *L. sporogenes.* These microorganisms have been found to be particularly effective in exhibiting antagonistic properties against undesirable strains of microorganisms, typically present or arisen in the article or on the surface (e.g, skin or urogenital zone of a human) the article is applied to, this typically upon prolonged period of time.

The microorganisms used herein have an activity such as to restrain the growth of undesirable strains of microorganisms or establishing of new undesirable species of microorganisms. Even some killing of microorganisms of undesirable species may occur. Indeed, these micro-organisms have the ability to inhibit pathogenic micro-organisms by competing for substrate. The antagonistic properties of the micro-organisms used herein is partly denoted their ability of producing different so called antimetabolites, such as lactic acid, lactoperoxidases, bacteriocins and carbon dioxides.

Advantageously the spore-forming lactic acid producing micro-organisms used herein, preferably the species *Lactobacillus sporogenes,* have the ability to create very quickly an environment that is not suitable for the growth of pathogens. This is due to the rapid growth, high yield and reproducibility of such micro-organisms in comparison to non-spore forming lactic acid producing bacteria like *Lactobacillus acidophilus.* Furthermore these micro-organisms are spore forming micro-organisms, i.e., they are able to survive longer and reproduce themselves in comparison to non spore-forming micro-organisms. In other words, by the formation of spores these micro-organisms can germinate and re-germinate in time sequence in line with the bodily fluid discharge (growth media) into an absorbent article, thereby ensuring long-lasting antagonistic properties against pathogens.

Without to be bound by theory, it is speculated herein that in the event where the bodily fluid discharge is reduced and hence less substrate is available for the micro-organisms, the micro-organisms used herein have the ability to be transformed in a dormant form, i.e., spore form, which will be activated again upon availability of further substrate, typically upon further bodily fluid discharge. Thus the present invention also allows to provide articles with effective antagonistic properties, e.g., odour controlling ability, while using reduced total amount of active material (typically odour control material). Indeed a reduced total amount of spore-forming lactic acid-producing micro-organisms (e.g. L. *sporogenes*) is needed to obtain a given odour control activity for a given disposable article, as compared to the total amount of non-spore forming antagonistic micro-organisms belonging to the genera Lactobacillus, Pediococcus, or Lactococcus that would be needed to get the same odour control activity.

Thus it is an advantage of the present invention to provide a disposable absorbent article, which can be worn for a relatively long period of time without pathogenic micro-organisms being allowed to grow to an extent in which undesirable odours are generated, in which the risk of infection is created and/or to an extent which will have a negative effect on skin. Advantageously the genetic identity, the lactic acid producing ability, the viability as well as the valuable antagonistic properties against pathogens are maintained even upon prolonged periods of use, typically during the whole wearing time of the article, e.g., from 1 hour to 10 hours.

A further advantage associated with the disposable absorbent articles of the present invention like pantiliners or pads, is a better feeling and more acceptable cleanness level in use. Indeed, it has been found that the microorganisms herein allow gelification of the bodily fluid discharge, thereby facilitating the fluid transport into the article. In a preferred embodiment the microorganisms are located in the core of the absorbent article, thereby changing the physical properties of the bodily fluid discharge. Such gelification will have the benefits of reducing the rewetting of the topsheet and the soiling through as might otherwise result from squeezing the article as a consequence of legs movement of a person wearing the article. This translates in consumer noticeable dryness and cleanness benefit. Thus the present invention provides a disposable absorbent article which combines an effective level of protection and long lasting odor control over a broad spectrum of malodor upon contact with bodily fluid, by using a single active material, the spore-forming lactic acid producing microorganisms, typically *L. sporogenes.*

Whereas the present invention is preferably directed to absorbent articles like pantiliners, feminine napkins, incontinent pads, diapers, tampons, interlabial pads, perspiration pads, surgical pads, breast pads, human or animal waste management devices and the like, other articles may include the micro-organisms as described herein too for the purpose of control or alleviation of pathogens, Indeed, other applications include other articles designed to be worn in contact with the external surface of a human or an animal such as clothing, bandages, thermal pads, acne pads, cold pads, compresses, surgical pads/dressings and the like, body cleansing articles like impregnated wipes (e.g. baby wipes, wipes for feminine intimate hygiene), articles for absorbing perspiration such as shoe insoles, and articles for animals like litters and the like.

### Summary of the Invention

The present invention relates to an article, preferably a disposable absorbent article, comprising spore-forming lactic acid producing microorganisms.

The present invention further encompasses the use, in an article, preferably a disposable absorbent article, of spore-forming lactic acid producing microorganisms, preferably *L. sporogenes,* for long lasting antagonistic properties against undesirable strains of microorganisms, typically for long lasting odour control of the article in use (for instance when worn in the urogenital zone).

### Detailed description of the Invention

By "article" it is meant herein any tridimentional solid material being able to receive/carry micro-organisms as described herein. By "article suitable for external application to a human or animal", it is meant any article suitable to be applied or worn in contact with the body of e.g., a human including especially the skin and the urogenital zone. Vaginal application (e.g., tampon) is considered external according to the present invention. The term 'disposable' is used herein to describe articles which are not intended to be launched or otherwise restored or reused as an article (i.e., they are intended to be discarded after a single use and, preferably to be recycled, composted or otherwise disposed of in an environmentally compatible manner).

Preferred articles according to the present invention are disposable absorbent articles that are designed to be placed against or in proximity to the body of a wearer and to absorb and/or contain fluids/exudates discharged from the body, such as disposable absorbent pantiliners, sanitary napkins, catamenials, incontinence inserts/pads, diapers, tampons, interlabial pads/inserts, breast pads, human or animal waste management devices and the like. Typically such human urine or faecal management devices comprise a bag having an aperture and a flange surrounding the aperture for preferably adhesive attachment to the urogential area and/or the perianal area of a wearer. Any faecal or urine management device known in the art is suitable for use herein. Such devices are described in for example WO 99/00084 to WO 99/00092. Other articles suitable according to the present invention also include other articles designed to be worn in contact with the body such as clothing, bandages, thermal pads, acne pads, cold pads, compresses, surgical pads/dressings and the like, articles for absorbing perspiration such as shoe insoles, shirt inserts, perspiration pads and the like, body cleansing articles like impregnated wipes (e.g. baby wipes, wipes for feminine intimate hygiene), impregnated tissues, towels, and the like, and articles for animals like litters and the like.

By "bodily fluids" it is meant herein any fluid produced by human or animal body occurring naturally or accidentally like for instance in the case of skin cutting, including for instance perspiration, urine, menstrual fluids, faeces, vaginal secretions and the like.

As an essential element the disposable article of the present invention comprises spore-forming lactic acid producing microorganisms. These microorganisms have the ability to survive in hostile environment in spore form (dormant form). Sporulation is the development in microorganisms of bodies each wrapped in a protective coat (a natural process of microencapsulation in a calcium-dipicolinic acid-peptidoglycan complex). Under favorable conditions, the spores germinate into viable bacilli (living form) and carry on their life activities. Suitable spore-forming lactic acid-producing microorganisms for use herein include the microorganisms belonging to the family Sporolactobacillaceae, particularly the genus Sporolactobacillus (e.g., *S. inulinus*). Sporolactobacillaceae combine the advantage of producing spores (also called endospores) which corresponds to the dormant form of the microorganisms together with the advantage of releasing antagonistic metabolites mainly lactic acid. Highly preferred spore-forming lactic acid producing microorganism for use herein is L. *sporogenes*

*L. sporogenes* was first isolated from green malt and described in 1933 by L.M. Horowitz-Wlassowa and N.W. Nowotelnow. It was submitted as L. *sporogenes* in the fifth edition (1939) of 'Bergey's manual of Determinative Bacteriology' as well as mentioned in recognized scientific publication, Korean J. Appl. Microb. & Bioengin. (1985) 13:185-190, J. Pharmaceut. Soc. Korea (1977) vol XXIII, 1-Feb, 473-474. *L. sporogenes* was transferred to *Bacillus* coagulans in the seventh edition of 'Bergey's manual of Determinative Bacteriology' due to simplification in cataloguing. However in honour of the original discoverers the name *L. sporogenes* is used widely. Reference is also made to the taxonomical classification of *Sporolactobacillus* in L'integratore Nutrizionale 2 (1) 1999, Stabilita' di integratori con Sporolactobacillus, classificazione tassonomica by L. Marossi and all.

According to the Eighth Edition of Bergey's Manual of Determinative Bacteriology, "various spore-bearing rods which produce lactic acid, are facultative or aerobic and catalase positive, have generally and correctly been assigned to the genus *Bacillus'.* The characteristics of *L. sporogenes* as cited in 'Bergey's Manual of Determinative Bacteriology' (seventh edition) and other sources are "gram positive spore-forming rods 0.9 by 3.0 to 5.0 micron size, aerobic to microaerophilic, producing L (+) lactic acid homofermentatively". *L. Sporogenes* also release other metabolites like carbon dioxide, diacetyl, bacteriocins, lacto-peroxidase.

Since *L. Sporogenes* exhibits characteristics typical of both genera *Lactobacillus* and *Bacillus,* its taxonomic position between the families *Lactobacillaceae* and *Bacillaceae* has often been discussed. This along with the fact that there is no universally accepted official classification leaves room for controversy in the nomenclature. More information about *L.sporogenes* is available from the commercial brochure 69/107, incorporated herein by reference, of Sochim International s.p.a. Milano, a supplier of the spore form of *L. Sporogenes.*

Some authors refer to *L.sporogenes* as *Bacillus coagulans. Bacillus coagulans* Hammer deposited as *Lactobacillus sporogenes* by Kabushiki Kaisha Naruse Fermentation Research Laboratory is commercially available under ATCC number 31284 (Internet information source : http://www.atcc.org/). ATCC stands for American Type Culture Collection.

The spore-forming lactic acid producing micro-organisms, especially *Lactobacillus sporogenes,* have the ability to create very quickly an environment that is not suitable for the growth of pathogens. This is due to the rapid growth, high yield and reproducibility of such micro-organisms in comparison to other lactic acid producing bacteria like *Lactobacillus acidophilus.* For example *L. sporogenes* needs 30 minutes for one generation while *L. acidophilus* needs 80 minutes. Furthermore the particularity of these micro-organisms is that these micro-organisms are spore forming lactic acid producing micro-organisms, i.e., they are able to survive longer and reproduce themselves in comparison to non spore-forming micro-organisms. Indeed in contrast to non-spore forming bacteria like L. *acidophilus, L sporogenes* are transformed in spores (dormant form) when the substrate is reduced (in absence or reduced amount of bodily fluid discharge) and germinate again upon further bodily fluid discharge, i.e., reproduce themselves again through the spore form. In other words, by the formation of spores these micro-organisms can germinate and re-germinate in time sequence in line with the bodily fluid discharge (growth media) into an absorbent article, thereby ensuring long-lasting antagonistic properties against pathogens.

Indeed *L. sporogenes* cells (in spore form) are protected from destruction by environmental factors by the naturally-present microencapsulation system, the spore coat. Once in the spore form the microorganism has the ability to be reactivated by environmental changes like pH and temperature changes and availability of substrate, typically by further bodily fluid discharge. For instance in use when the article is contacted with the skin or the urogenital zone of a wearer, substrates like humidity, transpiration and/or bodily fluid discharge are provided upon different sequence of time and hence the spores germination cycle will be in line with availability of the substrates. The optimum growth temperature range for these spores is between 30°C and 50°C and the optimum pH range is from 5.0 to 6.5. New fluid discharge like perspiration (pH 6), menstrual fluid discharge (pH 6.5), milk discharge (pH 6.4 ) and/or urine discharge (pH 6.4) will active the germination/re-germination of the spores. The spore coat imbibe water, swell and the increased water content will cause a rise in the metabolic rate of the sporulated bacilli. Outgrowths will begin to protrude from the spore-coats. The outgrown cells germinate and transform into viable vegetative cell (the microorganism in its 'living form'). The living form begin to proliferate multiplying rapidly. These micro-organisms continue their metabolic activities producing lactic acid and other metabolites which render the environmental non-conducive for the growth of harmful pathogenic micro-organisms. The germination process will be influenced by the body discharge in the article, leading to a somehow controlled germination process on demand. In other words all the spores will not germinate with the same kinetic but the germination will be in relation to the body fluid discharge on the article. This will contribute to sustain the effective beneficial microbial activity.

Typically the number of microorganisms per article, preferably per disposable absorbent article, exceeds 10⁴ cfu, preferably exceeds 10⁶ cfu, more preferably is from 10⁷ to 10¹² cfu.

Advantageously the microorganisms herein are easily cultivated, have a rapid growth, high yield and are readily handled at technical handling thereof. Furthermore in contrast to non-spore forming lactic acid producing microorganisms belong to the genera Lactobacillus and/or Pediococcus the microorganisms herein do not need continuous re-inoculation to maintain the microorganisms in a given media. Indeed they are able to re-inoculate themselves again through the spore form. These circumstances make the microorganisms herein also more easy to handle and thus also cheaper.

The microorganisms herein are typically used in a freeze-drying form. Their isolation process follows known routine processes for the isolation of pure cultures. The isolated pure cultures are then typed according to known methods, e.g., API. The microorganisms as described herein are then cultivated in a fermentor in a manner known per se, are separated from the medium using a separator or a centrifuge, are freeze-dried in a manner known per se and ground to a fine powder. The bacterial concentrate in powder so obtained is then typically mixed with a fermentable carbohydrate, e.g., glucose, to a desired concentration. Typically a final concentration of about 100 billion viable microorganisms per gram is obtained. Such powder is then ready for use in the absorbent article. Alternatively in some case it is possible to add the living bacteria to the absorbent article and then carry out a freeze drying thereof. Another available form is a lyophilized form.

In one embodiment herein, the articles, typical disposable absorbent articles are dried to a moisture content of less than 10%, preferably less than 5% and most preferably less than 1%, calculated as percentage of weight of the article.

Malodour is generated among other by inter or extra cellular metabolic activity which satisfy the bacteria needs for energy and proliferation. The metabolic activity aiming the production of vital oxygen, carbon and ammonium sources, leads to degraded odorous compounds as by products which are among low molecular weight of fatty acids, amines, mercaptan, indoles, ammonium, sulfides and the like. Bacteria that cause unpleasant smells may belong to, for example, the family Enterobacteriaceae, e.g., Proteus mirabilis, Proteus vulgaris, Escheriachia coli and Klebsiella.

Bacteria that cause urinal tract infections may belong to, for example, the families Enterobacteriaceae and Micrococcaceae or the genus Streptococcus. Examples of species and genera are Escherichia coli, Proteus mirabilis, Enterococcus, Klebsiella, Staphylococcus and Streptococcus.

Microorganisms associated with skin infections are Ascomycetes, Pseudomonadaceae and Micrococcaceae and the genus Streptococcus, e.g. Candida albicans, Pseudomonas, Staphylococcus and Streptococcus.

The particularities of the microorganisms herein is that they inhibit other microorganisms by competing for substrates, forming metabolites like L (+) form of lactic acid, enzymes like lacto-peroxidases, toxins, carbon dioxide, peroxides or antibiotics, so-called bacteriocines. They have the ability to sustain the growth and reduce the patogenicity of many pathogens like ones mentioned herein before and especially Proteus, Pseudomonas, Echerichia, Klebsiella, Enterococcus, Staphylococcus, Streptococcus and Candida.

The particularity of the microorganisms herein is that they may be naturally occurring microorganisms that are non-toxic and do not have any negative biological effect on humans. One advantage afforded by the use of antagonistic microorganisms is that there is avoided an undesired selection pressure on the micro environment, such as favoring potential decease-promoting microorganisms and therewith the risk of developing pathogenic strains that are resistant to antibiotics and chemopharmaceutical preparations. Since the antimicrobial system is based on a natural, biological process, there is less risk of environmental ecological and toxic disturbances.

It is speculated that the production of lactic acid lowers the pH to 4-5, thereby inhibiting the growth of putrefactive organisms like E.coli, which require a higher optimum pH (typically 6 to 7) for favourable growth conditions. Furthermore undissociated lactic acid has the tendency to penetrate the membrane of pathogens, lowering their intracellular pH and/or interfering with their metabolic processes such as oxidative phosphorylation, thereby inhibiting the growth of such pathogens

Other metabolites further contribute to inhibit the growth of pathogens. For example carbon dioxide is believed to reduce membrane permeability. Hydrogen peroxide / Lactoperoxidase are believed to oxidise basic proteins and to destroy the "enzymes factories" (ribosomes) of pathogens. Bacteriocins are proteins or protein complexes with bactericidal activity. Indeed, bacteriocins have the ability to link to particular receptors on the cell wall of microorganisms, thereby affecting the functionality of the cell wall/membranes. Bacteriocins are also able to affect DNA-synthesis and protein synthesis.

Advantageously it has now been found that the microorganisms herein have the advantage of changing the physical properties of bodily fluid. Indeed a gelification of the bodily fluid is obtained when the fluid comes into contact with the microorganisms herein. This results in reduced leakage/wet through of the absorbent articles as well as in improved dryness of the wearer facing surface. When talking about reduced leakage and/or improved dryness reference is made to a same absorbent article in absence of such microorganisms. As used herein the tospheet provides said wearer facing surface. Improved dryness and cleanness is particularly noticed on the wearer facing surface in those embodiments wherein the lactic acid producing microorganisms are located in the absorbent core.

Advantageously the presence of the micro-organisms according to the present invention provides absorbent articles which upon contact with bodily fluid, provide effective and prolonged antagonistic properties against pathogens, typically long lasting odor control, but also effective level of protection of the absorbent articles, resulting in consumer noticeable benefits like dryness and cleanness.

Without to be bound by theory, it is speculated that the cause of this gelification is the denaturation of the proteins. Indeed the microorganisms herein release lactic acid through glycogen fermentation. As proteins are sensitive to pH, the presence of lactic acid causes the soluble proteins contained into the bodily fluid to turn into insoluble form. This creates a sort of tri-dimensional net of molecules trapping globules, minerals, fats which results in the so called gelification of the bodily fluid.

### Optional agents

The articles according to the present invention may further comprise on top of the micro-organisms described herein before, other conventional agents or mixtures thereof. Although the primary benefits herein are obtained by using a single ingredient namely the spore forming lactic acid producing micro-organisms, further optimised benefit can be obtained by adding conventional agents as desired.

### Absorbent gelling materials

As is well-known from recent commercial practice, absorbent gelling materials (sometimes referred to as "super-sorbers") are becoming broadly used in absorbent articles. AGM's are materials which have fluid-absorbing properties.

Such materials are highly preferred herein due to their dual function of absorbing and retaining fluids and odors.

Such materials form hydrogels on contact with water (e.g., with urine, blood, and the like). One highly preferred type of hydrogel-forming, absorbent gelling material is based on polyacids, especially polyacrylic acid. Hydrogel- forming polymeric materials of this type are those which, upon contact with fluids (i.e., liquids) such as water or body fluids, imbibe such fluids and thereby form hydrogels. These preferred absorbent gelling materials will generally comprise substantially water-insoluble, slightly cross-linked, partially neutralized, hydrogel-forming polymer materials prepared from polymerizable, unsaturated, acid-containing monomers. In such materials, the polymeric component formed from unsaturated, acid-containing monomers may comprise the entire gelling agent or may be grafted onto other types of polymer moieties such as starch or cellulose. Acrylic acid grafted starch materials are of this latter type. Thus, the preferred absorbent gelling materials include hydrolyzed acrylonitrile grafted starch, acrylic acid grafted starch, polyacrylates, maleic anhydride-based copolymers and combinations thereof. Especially preferred absorbent gelling materials are the polyacrylates and acrylic acid grafted starch.

Whatever the nature of the polymer components of the preferred absorbent gelling materials, such materials will in general be slightly cross-linked. Crosslinking serves to render these preferred hydrogel-forming absorbent materials substantially water-insoluble, and cross-linking also in part determines the gel volume and extractable polymer characteristics of the hydrogels formed therefrom. Suitable cross-linking agents are well known in the art and include, for example, (1) compounds having at least two polymerizable double bonds; (2) compounds having at least one polymerizable double bond and at least one functional group reactive with the acid-containing monomer material; (3) compounds having at least two functional groups reactive with the acid-containing monomer materials; and (4) polyvalent metal compounds which can from ionic cross-linkages. Cross-linking agents of the foregoing types are described in greater detail in Masuda et al; U.S. Patent 4,076,663; Issued February 28, 1978. Preferred cross-linking agents are the di- or polyesters of unsaturated mono-or polycarboxylic acids with polyols, the bisacrylamides and the di-or triallyl amines. Especially preferred cross-linking agents are N,N'-methylenebisacrylamide, trimethylol propane triacrylate and triallyl amine. The cross-linking agent will generally comprise from about 0.001 mole percent to 5 mole percent of the preferred materials. More preferably, the cross-linking agent will comprise from about 0.01 mole percent to 3 mole percent of the gelling materials used herein.

The preferred, slightly cross-linked, hydrogel-forming absorbent gelling materials will generally be employed in their partially neutralized form. For purposes described herein, such materials are considered partially neutralized when at least 25 mole percent, and preferably at least 50 mole percent of monomers used to form the polymer are acid group-containing monomers which have been neutralized with a salt-forming cation. Suitable salt-forming cations include alkali metal, ammonium, substituted ammonium and amines. This percentage of the total monomers utilized which are neutralized acid group-containing monomers is referred to as the "degree of neutralization". Typically, commercial absorbent gelling materials have a degree of neutralization somewhat less than 90%.

The preferred absorbent gelling materials used herein are those which have a relatively high capacity for imbibing fluids encountered in the absorbent articles; this capacity can be quantified by referencing the "gel volume" of said absorbent gelling materials. Gel volume can be defined in terms of the amount of synthetic urine absorbed by any given absorbent gelling agent buffer and is specified as grams of synthetic urine per gram of gelling agent.

Gel volume in synthetic urine (see Brandt, et al, below) can be determined by forming a suspension of about 0.1-0.2 parts of dried absorbent gelling material to be tested with about 20 parts of synthetic urine. This suspension is maintained at ambient temperature under gentle stirring for about 1 hour so that swelling equilibrium is attained. The gel volume (grams of synthetic urine per gram of absorbent gelling material) is then calculated from the weight fraction of the gelling agent in the suspension and the ratio of the liquid volume excluded from the formed hydrogel to the total volume of the suspension. The preferred absorbent gelling materials useful in this invention will have a gel volume of from about 20 to 70 grams, more preferably from about 30 to 60 grams, of synthetic urine per gram of absorbent gelling material.

Another feature of the most highly preferred absorbent gelling materials relates to the level of extractable polymer material present in said materials. Extractable polymer levels can be determined by contacting a sample of preferred absorbent gelling material with a synthetic urine solution for the substantial period of time (e.g., at least 16 hours) which is needed to reach extraction equilibrium, by then filtering the formed hydrogel from the supernatant liquid, and finally by then determining the polymer content of the filtrate. The particular procedure used to determine extractable polymer content of the preferred absorbent gelling agent buffers herein is set forth in Brandt, Goldman and Inglin; U.S. Patent 4,654,039; Issues March 31,1987, Reissue 32,649, The absorbent gelling materials which are especially useful in the absorbent articles herein are those which have an equilibrium extractables content in synthetic urine of no more than about 17%, preferably no more than about 10% by weight of the absorbent gelling material.

The absorbent gelling materials herein before described are typically used in the form of discrete particles. Such absorbent gelling materials can be of any desired shape, e.g., spherical or semi-spherical, cubic, rod-like polyhedral, etc. Shapes having a large greatest dimension/smallest dimension ratio, like needles and flakes, are also contemplated for use herein. Agglomerates of absorbent gelling material particles may also be used.

The size of the absorbent gelling material particles may vary over a wide range. For reason of industrial hygiene, average particle sizes smaller than about 30 microns are less desirable. Particles having a smallest dimension larger than about 2mm may also cause a feeling of grittyness in the absorbent article, which is undesirable from a consumer aesthetics standpoint. Furthermore, rate of fluid absorption can be affected by particle size. Larger particles have very much reduced rates of absorption. Preferred for use herein are absorbent gelling material s particles substantially all of which have a particle size of from about 30 microns to about 2mm. "Particle Size" as used herein means the weighted average of the smallest dimension of the individual particles.

Typically, the amount of absorbent gelling material particles used in the articles herein, preferably the disposable absorbent articles, will range from 0 gm⁻² to 150gm⁻², preferably from 30gm⁻² to 110gm⁻², more preferably from 55gm⁻² to 85gm⁻².

### Odour control agents

Additional odour control agent or combinations thereof, known in the art for this purpose may be used herein. These agents can typically be classified according to the type of odour the agent is intended to combat. Odors may be chemically classified as being acidic, basic or neutral.

Alternatively, the odor control agents may be categorized with respect to the mechanism by which the malodor detection is reduced or prevented. For example, odor control agents which chemically react with malodorous compounds or with compounds which produce malodorous degradation products thereby generating compounds lacking odor or having an odor acceptable to consumers may also be utilized herein.

Suitable odor control agents for use herein typically include carbonates (e.g., sodium carbonate), bicarbonates (e.g., sodium bicarbonate), phosphates (e.g., sodium phosphate), sulphates (e.g., zinc and copper sulphates), carboxylic acids such as citric acid, lauric acid, boric acid, adipic acid and maleic acid, activated carbons, clays, zeolites, silicas and starches. Such odor control agents and systems are disclosed in more details hereinafter and for example in EP-A-348 978, EP-A- 510 619, WO 91/12029, WO 91/11977, WO 91/12030, WO 81/01643 and WO 96/06589.

Alternative odor control agents are ion exchange resins such as those described in US 4 289 513 and US 3340875.

Masking agents such as perfumes may also be used as odor control agents herein.

Typically, the articles herein may comprise an additional odor control agent or a mixture thereof at a level of from 0 gm⁻² to 600 gm⁻², preferably from 5 to 500 gm⁻², more preferably from 10 gm⁻² to 350 gm⁻² and most preferably from 20 gm⁻² to 200 gm⁻²

### The disposable articles

Preferred articles herein are disposable absorbent articles like pantiliners, feminine napkins, incontinent pads, diapers, nursing pads, and the like. The microorganisms as described herein (and optional absorbing gelling material and/or optional additional odor control agent(s)) may be incorporated into an article, preferably a disposable absorbent article by any of the methods disclosed in the art, for example layered on the core of the absorbent article or mixed within the fibres of the absorbent core.

The microorganisms as described herein and optional additional agents are preferably incorporated between two layers of cellulose tissue. Optionally the system may be bonded between two cellulose tissue layers with, for example, a hot melt adhesive or any suitable bonding system, as described in WO 94/01069.

In one embodiment of the present invention the microorganisms as described herein and optional additional agents are incorporated in a layered structure in accordance with the disclosure of WO 94/01069 or WO 95/17868. WO 95/17868 describes a layered structure substantially as described in WO 94/01069 with the exception that WO 95/17868 comprises a much higher quantity of absorbent gelling material in the intermediate layer which is between the fibrous layers (at least 120gm⁻²) that would be incorporated herein as an optional agent in the present invention. In the present invention the intermediate layer comprises a conventional glue as replacement for the polyethylene thermoplastic material. Conventional glues include for instance those commercially available from ATO Findley under the name H20-31® to glue the laminate layers and/or components together. Advantageously this method step allows to avoid the heating step necessary when using polyethylene powder.

Adhesive lines are preferably also placed on the edges of the laminate to ensure that the edges of the laminate stick and any loose spores and optional additional odor control agent present do not fall out of the laminate.

The microorganisms as described herein may be distributed homogeneously or non homogeneously over the entire absorbent article or in at least one layer of the topsheet or in at least one layer of the backsheet or in at least one layer of the core or any mixture thereof. The microorganisms as described herein may be distributed homogeneously or non homogeneously on the whole surface of the desired layer or layers, or on one or several area of the surface layer/layers to which it is positioned (e.g. central area and/or surrounding area like the edges of a layer of the absorbent article) or mixtures thereof. Preferably the microorganisms as described herein are located in the core (also called intermediate layer which is positioned between the topsheet and the backsheet). The presence of the microorganisms in the core is preferred because the core collects and absorbs bodily fluids. Thus the close proximity to the substrate contributes to improved antagonistic activity of the microorganisms. Indeed optimum inhibition of the degradation activity by putrefactive bacteria and proliferation of pathogens is obtained.

In one embodiment of the present invention the microorganisms as described herein are positioned such that at least a portion of the fluid discharge comes into contact with said microorganisms before the optional absorbent gelling material (e.g., AGM) and/or optional additional odour control agent if present. In particular, the microorganisms as described herein can be located in a separate layer from the optional absorbing gelling material and/or optional additional odor control agent if present. In such an embodiment the microorganisms as described herein are located towards the topsheet or in the topsheet itself (preferably the secondary topsheet) and the optional absorbing gelling material and/or optional additional odour control agent are located further away from the topsheet than the microorganisms. In one embodiment of the present invention, the microorganisms are positioned in at least one of the topsheet layers and the optional absorbing gelling material and/or optional additional odor control agent, are positioned in the core.

In another embodiment the microorganisms as described herein may be located in various layers, i.e. that the total amount of microorganisms is distributed in the topsheet layer and the core. The benefit of this execution are related with the combined action of microorganisms which inhibit the growth of degradative/pathogen bacteria in the core and on the body surface in contact with the absorbent article.

The microorganisms as described herein may be incorporated as a powder or a granulate (freeze-dried form or lyophilized form). When used in a granulate or particulate form the microorganisms as described herein and the optional absorbing gelling material and optional additional odor control agent may be granulated separately and then mixed together or granulated together.

The preferred absorbent article according to the present invention is described as follows:

### Absorbent core

According to the present invention, the absorbent can include the following components: (a) an optional primary fluid distribution layer preferably together with a secondary optional fluid distribution layer; (b) a fluid storage layer; (c) an optional fibrous ("dusting") layer underlying the storage layer; and (d) other optional components. According to the present invention the absorbent may have any thickness depending on the end use envisioned.

### a Primary/Secondary Fluid Distribution Layer

One optional component of the absorbent 'according to the present invention is a primary fluid distribution layer and a secondary fluid distribution layer. The primary distribution layer typically underlies the topsheet and is in fluid communication therewith. The topsheet transfers the acquired fluid to this primary distribution layer for ultimate distribution to the storage layer. This transfer of fluid through the primary distribution layer occurs not only in the thickness, but also along the length and width directions of the absorbent product. The also optional but preferred secondary distribution layer typically underlies the primary distribution layer and is in fluid communication therewith. The purpose of this secondary distribution layer is to readily acquire fluid from the primary distribution layer and transfer it rapidly to the underlying storage layer. This helps the fluid capacity of the underlying storage layer to be fully utilized. The fluid distribution layers can be comprised of any material typical for such distribution layers. In particular fibrous layers maintain the capillaries between fibers even when wet are useful as distribution layers.

### b Fluid Storage Layer

Positioned in fluid communication with, and typically underlying the primary or secondary distribution layers, is a fluid storage layer. The fluid storage layer can comprise any usual absorbent material or combinations thereof. It preferably comprises absorbent gelling materials in combination with suitable carriers.

Suitable carriers include materials which are conventionally utilized in absorbent structures such as natural, modified or synthetic fibers, particularly modified or non-modified cellulose fibers, in the form of fluff and/or tissues. Suitable carriers can be used together with the absorbent gelling material, however, they can also be used alone or in combinations. Most preferred are tissue or tissue laminates in the context of sanitary napkins and panty liners.

An embodiment of the absorbent structure made according to the present invention may comprise multiple layers comprises a double layer tissue laminate formed by folding the tissue onto itself. These layers can be joined to each other for example by adhesive or by mechanical interlocking or by hydrogen bridge bands. Absorbent gelling material or other optional material can be comprised between the layers.

Modified cellulose fibers such as the stiffened cellulose fibers can also be used. Synthetic fibers can also be used and include those made of cellulose acetate, polyvinyl fluoride, polyvinylidene chloride, acrylics (such as Orlon), polyvinyl acetate, non-soluble polyvinyl alcohol, polyethylene, polypropylene, polyamides (such as nylon), polyesters, bicomponent fibers, tricomponent fibers, mixtures thereof and the like. Preferably, the fiber surfaces are hydrophilic or are treated to be hydrophilic. The storage layer can also include filler materials, such as Perlite, diatomaceous earth, Vermiculite, etc., to improve liquid retention.

If the absorbent gelling material is dispersed non-homogeneously in a carrier, the storage layer can nevertheless be locally homogenous, i.e. have a distribution gradient in one or several directions within the dimensions of the storage layer. Non-homogeneous distribution can also refer to laminates of carriers enclosing absorbent gelling materials partially or fully.

### c Optional Fibrous ("Dusting") Layer

An optional component for inclusion in the absorbent core according to the present invention is a fibrous layer adjacent to, and typically underlying the storage layer. This underlying fibrous layer is typically referred to as a "dusting" layer since it provides a substrate on which to deposit absorbent gelling material in the storage layer during manufacture of the absorbent core. Indeed, in those instances where the absorbent gelling material is in the form of macro structures such as fibers, sheets or strips, this fibrous "dusting" layer need not be included. However, this "dusting" layer provides some additional fluid-handling capabilities such as rapid wicking of fluid along the length of the pad.

### d Other Optional Components of the absorbent structure

The absorbent core according to the present invention can include other optional components normally present in absorbent webs. For example, a reinforcing scrim can be positioned within the respective layers, or between the respective layers, of the absorbent core. Such reinforcing scrims should be of such configuration as to not form interfacial barriers to fluid transfer. Given the structural integrity that usually occurs as a result of thermal bonding, reinforcing scrims are usually not required for thermally bonded absorbent structures.

### The topsheet

According to the present invention the absorbent article comprises as an essential component a topsheet. The topsheet may comprise a single layer or a multiplicity of layers. In a preferred embodiment the topsheet comprises a first layer which provides the user facing surface of the topsheet and a second layer between the first layer and the absorbent structure/core.

The topsheet as a whole and hence each layer individually needs to be compliant, soft feeling, and non-irritating to the wearer's skin. It also can have elastic characteristics allowing it to be stretched in one or two directions. According to the present invention the topsheet may be formed from any of the materials available for this purpose and known in the art, such as woven and non woven fabrics and films. In a preferred embodiment of the present invention at least one of the layers, preferably the upper layer, of the topsheet comprises a hydrophobic, liquid permeable apertured polymeric film. Preferably, the upper layer is provided by a film material having apertures which are provided to facilitate liquid transport from the wearer facing surface towards the absorbent structure. If present the lower layer preferably comprises a non woven layer, an apertured formed film or an airlaid tissue.

### The backsheet

The backsheet primarily prevents the extrudes absorbed and contained in the absorbent structure from wetting articles that contact the absorbent product such as underpants, pants, pyjamas and undergarments. The backsheet is preferably impervious to liquids (e.g. menses and/or urine) and is preferably manufactured from a thin plastic film, although other flexible liquid impervious materials can also be used. As used herein, the term "flexible" refers to materials that are compliant and will readily conform to the general shape and contours of the human body. The backsheet also can have elastic characteristics allowing it to stretch in one or two directions.

The backsheet typically extends across the whole of the absorbent structure and can extend into and form part of or all of the preferred sideflaps, side wrapping elements or wings.

The backsheet can comprise a woven or noriwoven material, polymeric films such as thermoplastic films of polyethylene or polypropylene, or composite materials such as a film-coated nonwoven material. Preferably, the backsheet is a polyethylene film typically having a thickness of from about 0.012 mm (0.5 mil) to about 0.051 mm (2.0 mil).

Exemplary polyethylene films are manufactured by Clopay Corporation of Cincinnati, Ohio, under the designation P18-0401 and by Ethyl Corporation, Visqueen Division, of Terre Haute, Indiana, under the designation XP-39385. The backsheet is preferably embossed and/or matt finished to provide a more clothlike appearance. Further, the backsheet can permit vapors to escape from the absorbent structure, i.e. be breathable, while still preventing extrudates from passing through the backsheet. Also breathable backsheets comprising several layers, e.g. film plus non-woven structures, can be used.

The present invention is further illustrated by the following example.

### Examples:

### Example A

The feminine pads used in the following examples were Always (Always is a registered Trade Mark) as sold by the Procter & Gamble Company.

Each feminine pad was opened by cutting the wrap around the perforated coverstock at its bottom face approximately along a longitudinal edge of the release paper which covers the external adhesive layer. The side of the absorbent fibrous core was then exposed by slightly shifting the water impermeable plastic bottom layer and subsequently, the fibrous core was split into two halves, each having approximately the same thickness, along a plane which is parallel to the plane of the napkin itself. *L. sporogenes* powder was homogeneously distributed between these tow fibrous layers which were then joined together to reconstitute the absorbent core.

The water impermeable inner backsheet was then put back into its original position and the wrap around perforated coverstock was sealed along the cut by means of e.g. a double sided adhesive tape.
Samples were produced using the method above. *L. sporogenes* powder used was freeze/dried commercially available under ATCC number 31284 (ATCC= American Type Culture Collection). Each pad contained about 10^{*}10⁹ cfu of such microorganisms (ten billions cfu).

### Example B

Other pads were prepared by following the method in Example A except that absorbing gelling material (AGM) was added on top of the L. sporogenes spores in Example A. Accordingly the *L. sporogenes* and AGM were homogeneously distributed between these two fibrous layers which were then joined together to reconstitute the absorbent core.
*L. sporogenes* powder used was freeze-dried powder commercially available under ATCC number 31284 (ATCC= American Type Culture Collection). Each pad contained about 10^{*}10⁹ cfu of such micro-organisms. The AGM (0.8g) used was XZ 9589001, available from Dow Chemicals.

### Example C

Other pads were prepared by following the method in Example B except that after having split the fibrous core into two halves, *the L. sporogenes* powder was homogeneously distributed onto the upper halve fibrous layer (i.e., the fibrous layer halve intended to be closer to the topsheet) and AGM was homogeneously distributed onto the lower halve fibrous layer (i.e., the one intended to be closer to the backsheet of the pad once reconstituted). Then a layer of airlaid tissue (19 mm^{*} 70 mm of low basis weight) available from Fripa under the code/name NCB Tissue HWS was positioned between the two halve fibrous layers which are then joined together to reconstitute the absorbent core. The presence of the airlaid tissue between the two fibrous layer avoids direct contact between the *L. sporogenes* and AGM.
These samples were produced using freeze-dried powder of *L. sporogenes* commercially available under ATCC number 31284 (ATCC= American Type Culture Collection). The pads contained about 10^{*}10⁹ of such microorganisms.

All the above exemplified pads delivered outstanding odour control benefits for prolonged period of time when in use, typically when coming into contact with for example bodily fluids while delivering effective cleanness and dryness benefit. Also these pads were able to minimize the occurrence of skin problems.

## Claims

1. An article comprising a spore-forming lactic acid-producing microorganism.

2. An article according to claim 1 wherein said article is a disposable article, preferably a sanitary napkin, pantiliner, tampon, diaper, incontinent pad, breast pad, perspiration pad, human or animal waste management device or interlabial pad, or a body cleaning article, preferably a wipe for feminine intimate hygiene.

3. An article according to any of the preceding claims wherein said article is a disposable absorbent article comprising a liquid pervious topsheet, a backsheet and an absorbent core intermediate said backsheet and said topsheet.

4. An article according to any of the preceding claims wherein the microorganism is *Lactobacillus sporogenes.*

5. An article according to any of the preceding claims in which the number of such microorganisms per article exceeds 10⁴ cfu, preferably exceeds 10⁶ cfu, and more preferably is from 10⁷ to 10¹² cfu.

6. An article according to any of the preceding claims, which further comprises an absorbing gelling material or a mixture thereof.

7. An article according to claim 6, wherein the level of the absorbing gelling material or a mixture thereof is from 0 gm⁻² to 150gm⁻², preferably from 30gm⁻² to 110gm⁻², more preferably from 55gm⁻² to 85gm⁻².

8. An article according to any of the preceding claims, which further comprises an additional odour control agent or a mixture thereof.

9. An article according to claim 8, wherein the level of the additional odour control agent or a mixture thereof is from 0gm⁻² to 600gm⁻², preferably from 5gm⁻² to 500gm⁻², more preferably from 20gm⁻² to 200gm⁻².

10. The use, in an article, preferably a disposable absorbent article, of spore-forming lactic acid-producing micro-organisms, preferably *Lactobacillus sporogenes*, for long lasting antagonistic properties against undesirable strains of micro-organisms, preferably for long lasting odour control.
